# EUROPÄISCHE PATENTSCHRIFT

(11) **EP 0 346 269 B1**
(45) Veröffentlichungstag und Bekanntmachung des Hinweises auf die Patenterteilung: **13.10.1993**
(21) Anmeldenummer: 89730137.0
(22) Anmeldetag: 01.06.1989
(51) Int. Cl.: A61F 2/44

(54) **Endoprothese der Zwischenwirbelscheibe**
Intervertebral disk endoprothesis
Endoprothèse pour disque vertébral

(30) Priorität: 06.06.1988 DE 8807485 U
(43) Veröffentlichungstag der Anmeldung: 13.12.1989
(73) Patentinhaber: Fuhrmann, Gerhard, Dr., D-14167 Berlin (DE); Gross, Ulrich, Prof. Dr. med., D-12203 Berlin (DE); Kaden, Bertram, Dr., D-12157 Berlin (DE); Kranz, Curt, Dr.-Ing., D-10825 Berlin (DE); Schmitz, Herman-Josef, Dr. Dr., D-12165 Berlin (DE); Fritz, Thomas, D-13503 Berlin (DE)
(72) Erfinder: Fuhrmann, Gerhard, Dr., D-14167 Berlin (DE); Gross, Ulrich, Prof. Dr. med., D-12203 Berlin (DE); Kaden, Bertram, Dr., D-12157 Berlin (DE); Kranz, Curt, Dr.-Ing., D-10825 Berlin (DE); Schmitz, Herman-Josef, Dr. Dr., D-12165 Berlin (DE); Fritz, Thomas, D-13503 Berlin (DE)
(74) Vertreter: Christiansen, Henning, Dipl.-Ing.

(56) Entgegenhaltungen:
- EP-A- 0 277 282
- DE-A- 2 203 242
- US-A- 4 309 777
- SOVIET INVENTIONS ILLUSTRATED Allgemeine Sektion (P), Woche E 46, Zusammenfassung Nr. Q1081, P32, 5. Januar 1983, Derwent Publications Ltd., London, GB; & SU-A-895433 (KHARK ORTHOPAEDICS) 09.01.82

## Beschreibung

Die Erfindung betrifft eine Endoprothese der im Oberbegriff des Anspruchs 1 angegebenen Art.

Aus der DE-A-2 203 242 ist eine derartige Prothese bekannt.

Nachteilig ist dabei, daß das zwischen den Deckplatten angebrachte elastische Material nicht vollständig körperverträglich ist, da schädliche Langzeitreaktionen mit dem umgebenden Körpergewebe auf die Dauer nicht ausgeschlossen werden können.

Weiterhin ist aus der DE-A-3 529 761 eine Bandscheibenprothese bekannt, bei der zwischen zwei symmetrischen Abschlußplatten ein starres Distanzstück vorgesehen ist, welche durch das Zusammenwirken von ineinandergreifenden Paarungen von konvexen und konkaven Flächen gegeneinander beweglich sind.

Auch hierbei besteht der Nachteil, daß durch die aufeinander reibenden Flächen Abriebpartikel entstehen.

Der Erfindung liegt die Aufgabe zugrunde, eine Endoprothese der eingangs genannten Gattung anzugeben, bei der eine bessere Körperverträglichkeit gegeben ist und darüberhinaus auch verhindert wird, daß sich bei häufigen Wechselbelastungen Materialpartikel ablösen und vagabundieren.

Diese Aufgabe wird mit den kennzeichnenden Merkmalen des Anspruchs 1 gelöst.

Die Erfindung beruht auf der Erkenntnis, daß eine das viskoelastische Material umgebende Manschette in Form eines Wellrohrs, das bevorzugt aus Titan besteht, einerseits eine körperverträgliche Außenoberfläche und andererseits einen hermetisch dichtenden Einschluß bildet.

Das Wellrohr ist dabei bevorzugt in die elastischen Eigenschaften in der Weise einbezogen, daß es ein übermäßiges Ausbauchen des Innenkörpers bei Stauchung verhindert und somit zur Formstabilität beiträgt. Insbesondere wird durch die Unterdrückung des Ausbauchens auch das schmerzhafte Berühren benachbarter Nerven unterbunden.

Durch Auffüllung des viskoelastischen Materials ganz oder teilweise mit gerichteten oder ungerichteten Fasern läßt sich die Viskosität des elastischen Innenkörpers steuern. Durch die Erhöhung des Auffüllungsgrades verringert sich die Kompressibilität. Bei Belastung krümmt sich die Anordnung die senkrecht auf der Belastungsrichtung und senkrecht auf der Richtung des Gradienten der Füllungsgradänderung steht. Durch geeignete Verteilung, beispielsweise durch eine Vergrößerung des Füllungsgrades im Zentrum läßt sich auch eine Anordnung erzeugen, bei der die Stirnflächen eine Art "Wippe" mit allseitiger Neigungsmöglichkeit bilden. Bei unsymmetrischer Anordnung der Füllung läßt sich auch ein unsymmetrisches bevorzugtes Kipp- oder Schubverhalten erzeugen.

Das das Wellrohr ausfüllende viskoelastische Material besteht insbesondere aus polymerisierbarem Material, das bei der Herstellung flüssig in eine Einfüllöffnung eingebracht wird. Zusätzlich ist vorteilhafterweise auch noch eine Entlüftungsöffnung vorgesehen. Als viskoelastisches Material eignet sich insbesondere Silikonkleber, wie er für das Verkleben von Blutgefäßen verwendet wird.

Die Anwendung der erfindungsgemäßen Endoprothese erfolgt bevorzugt in der Weise, daß die benachbarten Wirbel abgefräst werden, so daß die dann freiliegende Spongiosa in Bereiche der mit einer porösen und insbesondere bioaktiven Oberfläche versehenen Deckplatten einwächst.

Zur Befestigung von Deckplatten und Wellrohr sind einschraubbare Klemmeinrichtungen oder Schweißverbindungen günstig. Auch eine Verbindung mittels eines Spannrings ist vorteilhaft.

Gemäß einer ersten vorteilhaften Ausführungsform besteht die Endoprothese der Zwischenwirbelscheibe aus einem geschlossenen Federkörper, der innen mit viskoelastischem, unterschiedlich faserverstärktem Material gefüllt ist und an beiden Enden mit ebenen Deckplatten aus bioaktivem Material dichtend und fest verbunden ist.

Bei einer anderen Ausführungsvariante besteht die Endoprothese der Zwischenwirbelscheibe aus einem geschlossenen Federkörper, der innen mit viskoelastischem, räumlich unterschiedlich faserverstärktem Material gefüllt ist und an beiden Enden mit starren Deckplatten dichtend und fest verbunden ist, die den Wirbelkörpern angepaßte Oberflächen und seitwärts angeordnete Laschen zur Aufnahme von als Zugschrauben wirkenden Befestigungsschrauben haben.

Vorteilhafte Weiterbildungen der Erfindung sind in den Unteransprüchen gekennzeichnet bzw. werden nachstehend zusammen mit der Beschreibung der bevorzugten Ausführung der Erfindung anhand der Figuren näher dargestellt. Es zeigen:
Figur 1 ein erstes Ausführungsbeispiel der Erfindung in vergrößerter Seitenansicht,
Figur 2 ein zweites Ausführungsbeispiel in einer entsprechenden Darstellung sowie
Figuren 3 und 4 vergrößerte Details von Ausführungsvarianten.

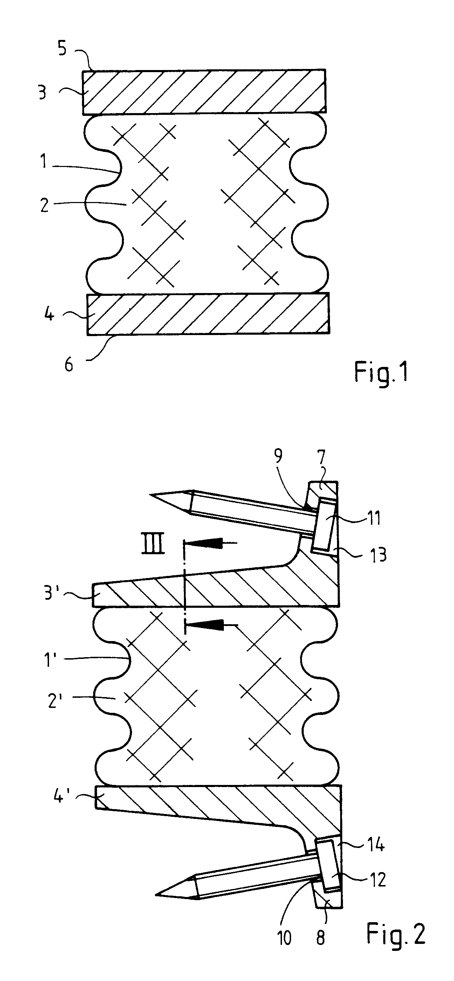

Bei dem ersten in Figur 1 dargestellten Ausführungsbeispiel besteht die Endoprothese der Zwischenwirbelscheibe aus einem geschlossenen von einem Wellrohr 1 umgebenen Federkörper, der innen mit viskoelastischem Material 2 gefüllt ist und an beiden Enden mit ebenen Deckplatten 3, 4 aus bioaktivem oder mit einer bioaktiven Beschichtung versehenem Material dichtend und fest verbunden ist. Das Wellrohr ist grundsätzlich den entsprechenden Federelementen ähnlich, wie sie in barometrischen Druckmeßdosen oder dergleichen verwendet werden.

Das viskoelastische Material besteht bevorzugt aus körperverträglichem Silikon. Damit bildet der Federkörper ein stabiles Pufferelement, welches sich komprimieren und verformen läßt, ohne daß sich der axiale Querschnitt wesentlich ändert, da dieser durch das flexible Wellrohr umfaßt ist. Neben Kompressionsbewegungen sind aber Kipp-, Scher-und in beschränktem Maße auch Torsionsbewegungen möglich, ohne daß sich der Querschnitt des Federkörpers vergrößert. Der Federkörper ist bezüglich seiner Oberflächeneigenschaften beständig und unterliegt keiner Abnutzung. Um die Implantate an spezielle Anforderungen anzupassen, ist vorgesehen, die Elastiztätseigenschaften des Materials 2 und gegebenenfalls auch des Wellrohrs 1 lokal unterschiedlich auszugestalten. Dabei ist für das Material 2 eine variable Faserverstärkung vorgesehen, welche die Kompressibilität örtlich verändert, so daß bei Kompression ein "Abrollen" auf dem Bereich mit Faserfüllung stattfindet. Durch lokale Schwächung des die Füllung ummantelnden Wellrohrs läßt sich dieser durch die Inhomogenität des Federkörpers bedingte Effekt noch verstärken. Die Außenflächen 5 und 6 der Deckplatten 3 und 4 sind entweder der Form der benachbarten natürlichen Wirbelkörper angepaßt oder aber eben ausgebildet. Die Flächen 5 und 6 weisen eine - nicht näher dargestellte - bioaktive Beschichtung auf, welche das Anwachsen von Körpergewebe begünstigt. Derartige Beschichtungen sind Hydroxyl-Apatit, keramisches HIP-Material oder Polylactid. Resorbierbares Material wird dabei bevorzugt in Sekundär-Poren eingebracht, an das der Knochen anwächst, nachdem das resorbierbare Material abgebaut ist. Auf diese Weise werden auch Infektionen unterdrückt. Eine poröse Oberfläche des metallischen Grundkörpers der Deckplatten 3 und 4 läßt sich beispielsweise durch Sandstrahlen erreichen. Das Anwachsen der Wirbelkörper wird begünstigt durch ein Anfräsen der Knochenoberfläche, so daß die Spongiosa freigelegt ist und eine der Fläche 5 bzw. 6 angepaßte - vorzugsweise ebene - Kontaktfläche des Knochens zur Verfügung steht. Die metallischen Teile des Implantats bestehen einheitlich aus Titan (oder aus einem anderen geeigneten Werkstoff wie für Implantationszwecke geeignetem rostfreiem Stahl), um eine größtmögliche Körperverträglichkeit zu sichern und Korrosion auszuschließen.

Bei dem zweiten in Figur 2 wiedergegebenen Ausführungsbeispiel besteht die Endoprothese der Zwischenwirbelscheibe ebenfalls aus einem geschlossenen von einem Wellrohr 1' umgebenen Federkörper, der innen mit viskoelastischem, unterschiedlich faserverstärktem Material 2' gefüllt ist und an beiden Enden mit starren Deckplatten 3', 4' dichtend und fest verschlossen ist. Mit den Deckplatten 3' und 4' sind seitwärts angeordnete laschenförmige Flansche 7, 8 verbunden, die senkrecht zu den Flächen der Deckplatten gerichtet sind. Die Flansche sind mit Löchern 9 und 10 zum Durchlassen von Knochenschrauben 11, 12 versehen, die so gerichtet sind, daß sich die in die Löcher von außen her eingefügten Schrauben 11 und 12 in Einschraubrichtung aufspreizen und somit Zugschrauben für die Deckplatten 3' und 4' bilden. Die Löcher 9 und 10 weisen Erweiterungen 13 und 14 auf, die die Köpfe üblicher Knochenschrauben aufnehmen, so daß diese darin versenkbar sind. Mittels der Flansche 7 und 8 und der Knochenschrauben 11 und 12 werden die Implantate mit den Wirbelkörpern von vorn her verschraubt. Auch die Knochenschrauben bestehen aus dem metallischen Werkstoff des übrigen Implantats.

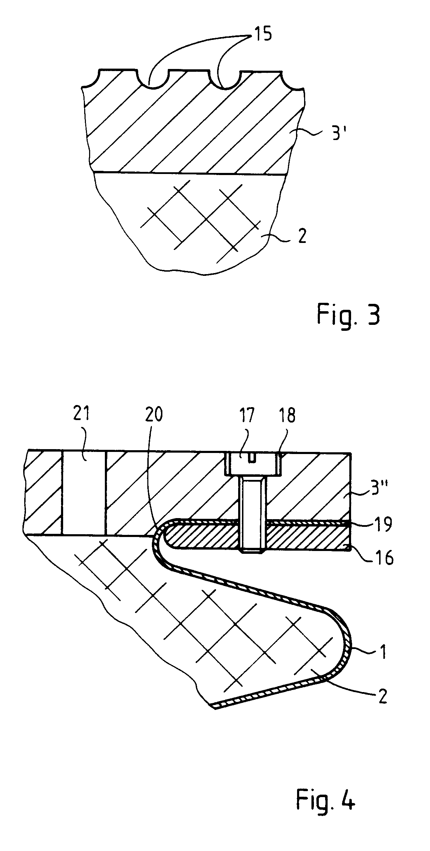

Bei der in Figur 3 wiedergegebenen Ausführungsvariante handelt es sich um ein vergrößertes Detail des Ausführungsbeispiels gemäß Figur 2 entsprechend dem dort mit III bezeichneten Schnitt. In die Deckplatte 3' sind Nuten 15 eingelassen, welche Führungen beim Kontakt mit dem benachbarten Wirbel bilden und für eine erhöhte Festigkeit der Verbindung beim Anziehen mit den Zugschrauben 11 und 12 gemäß Figur 2 sorgen.

In Figur 4 ist ein Teil einer anderen Ausführungsvariante wiedergegeben, welche beispielsweise eine Weiterbildung des in Figur 1 dargestellten Ausführungsbeispiels bildet.

Hier ist das Wellrohr 1 mit der Deckplatte 3" mittels eines Klemmrings 16 verbunden, wobei der Klemmring als geteilter Ring ausgebildet ist, in dessen Gewinde eine von der Außenseite der Deckplatte her eingesetzte Schraube 17 eingreift, deren Kopf sich in einer entsprechenden, den Kopf der Schraube aufnehmenden Ausnehmung 18 abstützt. Der Randbereich 19 des Wellrohrs 1 wird somit zwischen der Unterseite der Deckplatte 3", die dort einen dem Klemmring 16 teilweise als Ausnehmung angeformten Bereich 20 besitzt, eingespannt und läßt sich somit einfach montieren. Die Festigkeit der Verbindung ist bei allen zu erwartenden Beanspruchungen gewährleistet.

Eine Durchlaßöffnung 21 in der Deckplatte 3" dient zum Befüllen des Inneren mit viskoelastischem Material - oder zusammen mit einer gleichartigen Öffnung als Entlüftung.

Die Erfindung beschränkt sich in ihrer Ausführung nicht auf das vorstehend angegebene bevorzugte Ausführungsbeispiel. Vielmehr ist eine Anzahl von Varianten denkbar, welche von der dargestellten Lösung auch bei grundsätzlich anders gearteten Ausführungen Gebrauch machen.

## Patentansprüche

1. Endoprothese der Zwischenwirbelscheibe, die mit elastischem Material (2) gefüllt und mit stirnseitigen Deckplatten (3, 4) versehen ist,
**gekennzeichnet durch**
ein kreisförmiges oder elliptisches das viskoelastische Material (2) umgebendes Wellrohr (1), das durch die Deckplatten (3, 4) abgeschlossen ist.

2. Endoprothese nach Anspruch 1, **dadurch gekennzeichnet,** daß das Wellrohr (1) aus bioverträglichem Metall, insbesondere rostsicherem Stahl oder Titan, oder Kunststoff besteht.

3. Endoprothese nach Anspruch 1 oder 2, **dadurch gekennzeichnet,** daß das viskoelastische Material (2) ganz oder teilweise mit gerichteten oder ungerichteten Fasern gefüllt ist.

4. Endoprothese nach Anspruch 3, **dadurch gekennzeichnet,** daß die Füllung inhomogen ausgebildet ist, insbesondere derart, daß sich in Richtung auf mindestens einen Randbereich hin die Kompressibilität vergrößert.

5. Endoprothese nach einem der vorangehenden Ansprüche, **dadurch gekennzeichnet,** daß das viskoelastische Material (2) aushärtbare, ursprünglich flüssige Bestandteile, insbesondere Silikon-Kleber, aufweist.

6. Endoprothese nach Anspruch 5, **dadurch gekennzeichnet,** daß mindestens eine der Deckplatten und/oder das Wellrohr mindestens eine verschließbare Einfüll- und/oder Lüftungsöffnung (21) aufweist.

7. Endoprothese nach einem der vorangehenden Ansprüche, **dadurch gekennzeichnet,** daß das Wellrohr mittels einer schraubbaren Klemmeinrichtung an mindestens einer Deckplatte befestigt ist.

8. Endoprothese nach Anspruch 7, **dadurch gekennzeichnet,** daß die Klemmeinrichtung aus einem Klemmring (16) besteht.

9. Endoprothese nach einem der Ansprüche 1 bis 4, **dadurch gekennzeichnet,** daß das Wellrohr mit mindestens einer Deckplatte verschweißt ist.

10. Endoprothese nach einem der Ansprüche 1 oder 2, **dadurch gekennzeichnet,** daß das Wellrohr eine in Quer- oder Längsrichtung veränderliche Wandstärke aufweist.

11. Endoprothese nach einem der vorangehenden Ansprüche, **dadurch gekennzeichnet,** daß mindestens eine der nach außen weisenden Stirnflächen der Deckplatten Dorne, Radial- und/oder Umfangsnuten (15) aufweist.

12. Endoprothese nach einem der vorangehenden Ansprüche, **dadurch gekennzeichnet,** daß mindestens eine der nach außen weisenden Stirnflächen der Deckplatten und/oder des Wellrohrs eine oberflächenvergrößernde, insbesondere poröse, Beschichtung oder Oberflächengestaltung bzw. entsprechende Bereiche aufweist.

13. Endoprothese nach einem der vorangehenden Ansprüche, **dadurch gekennzeichnet,** daß mindestens eine der nach außen weisenden Stirn- und/oder Seitenoberflächen Einlagerungen aus bioaktivem Material aufweist.

14. Endoprothese nach Anspruch 13, **dadurch gekennzeichnet,** daß die bioaktive Fläche aus Hydroxyl-Apatit, Polylactid und/oder HIP-Material besteht.

15. Endoprothese nach einem der vorangehenden Ansprüche, **dadurch gekennzeichnet,** daß mindestens eine der Deckplatten einen Flansch (7, 8) aufweist.

16. Endoprothese nach Anspruch 15, **dadurch gekennzeichnet,** daß der Flansch sich parallel zur Mittelachse des Wellrohres (1') erstreckt.

17. Endoprothese nach Anspruch 16, **dadurch gekennzeichnet,** daß der Flansch (7, 8) eine Bohrung (9, 10) aufweist.

18. Endoprothese nach Anspruch 17, **dadurch gekennzeichnet,** daß die Richtung der Bohrung (9, 10) sich zur Mittelachse des Wellrohres (1') von der Deckplatte (3', 4') entfernt, so daß eine die Bohrung durchquerende Schraube (11, 12) eine Zugschraube bildet.

19. Endoprothese nach Anspruch 17, **dadurch gekennzeichnet,** daß die Bohrung eine Ausnehmung (13, 14) für einen Schraubenkopf an der von der Mittelachse des Wellrohres (1') abgewandten Seite aufweist.

20. Endoprothese nach einem der Ansprüche 15 bis 19, **dadurch gekennzeichnet,** daß die Außenfläche der Deckplatte (3') Nuten (13) aufweist, welche senkrecht zum Flansch gerichtet sind.

21. Endoprothese nach einem der vorangehenden Ansprüche, **dadurch gekennzeichnet,** daß mindestens eine der Deckplatten an ihrer Außenseiten eine Formgebung aufweist, welche an die Stirnfläche eines benachbarten Wirbels angepaßt ist.

## Claims

1. Intervertebral disc endoprosthesis which is filled with elastic material (2) and provided with cover plates (3,4) at its end faces, characterised by a circular or elliptical corrugated tube (1) surrounding the viscoelastic material (2) and closed off by the cover plates (3,4).

2. Endoprosthesis according to claim 1, characterised in that the corrugated tube (1) consists of bioacceptable metal, particularly stainless steel or titanium, or plastics.

3. Endoprosthesis according to claim 1 or 2, characterised in that the viscoelastic material (2) is wholly or partly filled with aligned or unaligned fibres.

4. Endoprosthesis according to claim 3, characterised in that the filling is non-homogeneous, particularly such that its compressibility increases towards at least one edge area.

5. Endoprosthesis according to one of the preceding claims, characterised in that the viscoelastic material (2) contains curable, originally liquid components, particularly silicone adhesives.

6. Endoprosthesis according to claim 5, characterised in that at least one of the cover plates and/or the corrugated tube has at least one sealable opening (21) for filling and/or ventilating.

7. Endoprosthesis according to one of the preceding claims, characterised in that the corrugated tube is attached to at least one cover plate by means of a screwable clamping device.

8. Endoprosthesis according to claim 7, characterised in that the clamping device comprises a clamping ring (16).

9. Endoprosthesis according to one of claims 1 to 4, characterised in that the corrugated tube is welded to at least one cover plate.

10. Endoprosthesis according to one of claims 1 or 2, characterised in that the corrugated tube has a wall thickness which is variable in the transverse or longitudinal direction.

11. Endoprosthesis according to one of the preceding claims, characterised in that at least one of the outwardly facing end faces of the cover plates has spikes or radial and/or circumferential grooves (15).

12. Endoprosthesis according to one of the preceding claims, characterised in that at least one of the outwardly facing end faces of the cover plates and/or of the corrugated tube has a coating or surface structure which enlarges the surface area and, more particularly, is porous, or has areas of this nature.

13. Endoprosthesis according to one of the preceding claims, characterised in that at least one of the outwardly facing end and/or side faces has inlays of bioactive material.

14. Endoprosthesis according to claim 13, characterised in that the bioactive area consists of hydroxyl apatite, polylactide and/or HIP material.

15. Endoprosthesis according to one of the preceding claims, characterised in that at least one of the cover plates has a flange (7,8).

16. Endoprosthesis according to claim 15, characterised in that the flange extends parallel to the central axis of the corrugated tube (1').

17. Endoprosthesis according to claim 16, characterised in that the flange (7,8) has a bore (9,10).

18. Endoprosthesis according to claim 17, characterised in that the direction of the bore (9,10) moves away from the cover plate (3',4') towards the central axis of the corrugated tube (1'), so that a screw (11,12) passing through the bore forms a tension screw.

19. Endoprosthesis according to claim 17, characterised in that the bore has a recess (13,14) for a screw head on the side remote from the central axis of the corrugated tube (1').

20. Endoprosthesis according to one of claims 15 to 19, characterised in that the outer surface of the cover plate (3') has grooves (13) directed perpendicularly to the flange.

21. Endoprosthesis according to one of the preceding claims, characterised in that at least one of the cover plates has, on its outer surfaces, a shape which is adapted to the end face of an adjacent vertebra.

## Revendications

1. Endoprothèse de disque intervertébral, emplie de matériau élastique (2) et dotée de plaques de recouvrement (3, 4) frontales, caractérisée par un tube ondulé (1) circulaire ou elliptique entourant le matériau viscoélastique (2) et obturé par les plaques de recouvrement (3, 4).

2. Endoprothèse selon la revendication 1, caractérisée en ce que le tube ondulé (1) est en un métal biocompatible, notamment acier inoxydable ou titane, ou en matière plastique.

3. Endoprothèse selon la revendication 1 ou 2, caractérisée en ce que le matériau viscoélastique (2) est empli totalement ou partiellement de fibres orientées ou non orientées.

4. Endoprothèse selon la revendication 3, caractérisée en ce que le remplissage est hétérogène, notamment de telle sorte que la compressibilité s'accroît dans la direction d'au moins une zone marginale.

5. Endoprothèse selon une des revendications précédentes, caractérisée en ce que le matériau viscoélastique (2) présente des constituants durcissables, originellement liquides, notamment une colle de silicone.

6. Endoprothèse selon la revendication 5, caractérisée en ce qu'au moins une des plaques de recouvrement et/ou le tube ondulé présente au moins un orifice de remplissage et/ou de désaération (21) obturable.

7. Endoprothèse selon une des revendications précédentes, caractérisée en ce que le tube ondulé est fixé à au moins une plaque de recouvrement au moyen d'un dispositif de serrage vissable.

8. Endoprothèse selon la revendication 7, caractérisée en ce que le dispositif de serrage se compose d'une bague de serrage (16).

9. Endoprothèse selon une des revendications 1 à 4, caractérisée en ce que le tube ondulé est soudé à au moins une plaque de recouvrement.

10. Endoprothèse selon une des revendications 1 ou 2, caractérisée en ce que le tube ondulé présente une épaisseur de paroi variable dans la direction transversale ou longitudinale.

11. Endoprothèse selon une des revendications précédentes, caractérisée en ce qu'au moins une des faces frontales, dirigées vers l'extérieur, des plaques de recouvrement présente des broches, des rainures radiales et/ou des rainures circonférentielles (15).

12. Endoprothèse selon une des revendications précédentes, caractérisée en ce qu'au moins une des faces frontales, dirigées vers l'extérieur, des plaques de recouvrement et/ou du tube ondulé présentent un revêtement ou une configuration superficielle agrandissant la surface, notamment poreux, ou des zones correspondantes.

13. Endoprothèse selon une des revendications précédentes, caractérisée en ce qu'au moins une des faces frontales et/ou latérales dirigées vers l'extérieur présente des inclusions en matériau bioactif.

14. Endoprothèse selon la revendication 13, caractérisée en ce que la face bioactive est en hydroxyapatite, ou Polylactid et/ou matériau à compression isostatique à températures élevées.

15. Endoprothèse selon une des revendications précédentes, caractérisée en ce qu'au moins une des plaques de recouvrement présente une bride (7, 8).

16. Endoprothèse selon la revendication 15, caractérisée en ce que la bride s'étend parallèlement à l'axe médian du tube ondulé (1').

17. Endoprothèse selon la revendication 16, caractérisée en ce que la bride (7, 8) présente un trou (9, 10).

18. Endoprothèse selon la revendication 17, caractérisée en ce que la direction du trou (9, 10) s'éloigne de la plaque de recouvrement (3', 4') par rapport à l'axe médian du tube ondulé (1'), de sorte qu'une vis (11, 12) traversant le trou forme une vis de tension.

19. Endoprothèse selon la revendication 17, caractérisée en ce que le trou présente un évidement (13, 14) destiné à une tête de vis sur la face opposée à l'axe médian du tube ondulé (1').

20. Endoprothèse selon une des revendications 15 à 19, caractérisée en ce que la surface externe de la plaque de recouvrement (3') présente des rainures (13) qui sont perpendiculaires à la bride.

21. Endoprothèse selon une des revendications précédentes, caractérisée en ce qu'au moins une des plaques de recouvrement présente sur ses faces externes un modelage adapté à la face frontale d'une vertèbre voisine.
